(19) Europäisches Patentamt
European Patent Office
Office européen des brevets

(11) Veröffentlichungsnummer: 0 384 230 A1

(12) **EUROPÄISCHE PATENTANMELDUNG**

(21) Anmeldenummer: 90102525.4

(22) Anmeldetag: 09.02.90

(51) Int. Cl.⁵: C07C 323/62, C07C 317/44, C07C 59/68, C07C 69/65, C07C 323/20, C07C 43/29, C07C 317/22, A01N 37/38

(30) Priorität: 23.02.89 DE 3905518

(43) Veröffentlichungstag der Anmeldung: 29.08.90 Patentblatt 90/35

(84) Benannte Vertragsstaaten: BE CH DE DK ES FR GB IT LI NL SE

(71) Anmelder: BAYER AG
D-5090 Leverkusen 1 Bayerwerk(DE)

(72) Erfinder: Haug, Michael, Dr.
Fahner Weg 5
D-5060 Bergisch Gladbach 2(DE)
Erfinder: Marhold, Albrecht, Dr.
Carl-Duisberg-Strasse 329
D-5090 Leverkusen 1(DE)
Erfinder: Santel, Hans-Joachim, Dr.
Grünstrasse 9a
D-5090 Leverkusen 1(DE)
Erfinder: Lürssen, Klaus, Dr.
August-Kierspel-Strasse 145
D-5060 Bergisch Gladbach 2(DE)
Erfinder: Schmidt, Robert R., Dr.
Im Waldwinkel 110
D-5060 Bergisch Gladbach 2(DE)

# (54) Substituierte Phenoxyphenylpropionsäure-Derivate.

(57) Die Erfindung betrifft neue substituierte Phenoxyphenylpropionsäure-Derivate der Formel (I)

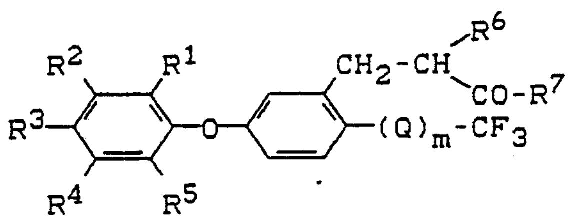

(I)

in welcher
m für die Zahlen 0 oder 1 steht,
Q für S, SO oder $SO_2$ steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Cyano, Carboxy, für gegebenenfalls durch Halogen substituiertes Alkyl oder für Alkoxycarbonyl steht,
$R^7$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^8$ steht, und

EP 0 384 230 A1

$R^8$ die in der Beschreibung angegebene Bedeutung hat,
Verfahren und neue Zwischenprodukte zu ihrer Herstellung sowie ihre Verwendung als Herbizide.

## Substituierte Phenoxyphenylpropionsäure-Derivate

Die Erfindung betrifft neue substituierte Phenoxyphenylpropionsäure-Derivate, Verfahren und neue Zwischenprodukte zu ihrer Herstellung sowie ihre Verwendung als Herbizide.

Es ist bekannt, daß bestimmte Phenoxyphenylverbindungen, wie z. B. 3-(2,4-Dichlor-phenoxy)-6-nitro-benzoesäuremethylester(Bifenox)herbizid wirksam sind (vergl. US-P 3 652 645 und US-P 3 776 715). Die Wirkung dieser bekannten Verbindung ist jedoch nicht in allen Belangen zufriedenstellend.

Es wurden nun neue substituierte Phenoxyphenylpropionsäure-Derivate der allgemeinen Formel (I)

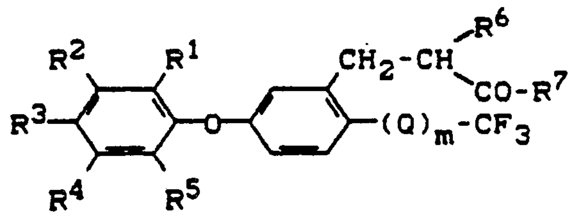

(I)

in welcher

Q für S, SO oder $SO_2$ steht,

m für die Zahlen 0 oder 1 steht,

$R^1$ für Wasserstoff, Halogen, Cyano oder Trifluormethyl steht,

$R^2$ für Wasserstoff oder Halogen steht,

$R^3$ für Halogen, Cyano, Trifluormethyl, Trifluormethoxy oder Trifluormethylsulfonyl steht,

$R^4$ für Wasserstoff oder Halogen steht,

$R^5$ für Wasserstoff oder Halogen steht,

$R^6$ für Wasserstoff, Cyano, Carboxy, für gegebenenfalls durch Halogen substituiertes Alkyl oder für Alkoxycarbonyl steht,

$R^7$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^8$ steht, worin

$R^8$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-Äquivalent steht oder für die Gruppierung

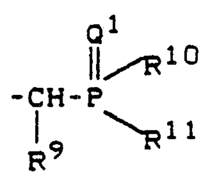

steht, worin

$R^9$ für Wasserstoff, Alkyl, Aryl, Furyl, Thienyl oder Pyridyl steht,

$R^{10}$ für Alkyl oder Alkoxy steht,

$R^{11}$ für Alkoxy steht und

$Q^1$ für Sauerstoff oder Schwefel steht, oder

$R^8$ für die Gruppierung $-(CH_2)_n-R^{12}$ steht, worin

n für die Zahlen 0, 1 oder 2 steht und

$R^{12}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht, gefunden.

Weiter wurde gefunden, daß man die neuen substituierten Phenoxyphenylpropionsäure-Derivate der

allgemeinen Formel (I) erhält, wenn man

(a) substituierte Phenoxybenzylhalogenide der allgemeinen Formel (II)

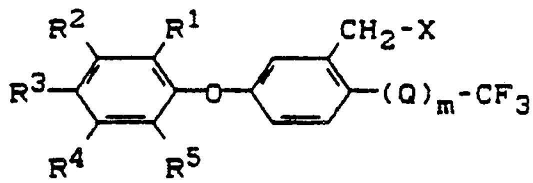

(II)

in welcher
Q, $R^1$, $R^2$, $R^3$, $R^4$, $R^5$ und m die oben angegebenen Bedeutungen haben und
X für Halogen steht,
mit Carbonylverbindungen der allgemeinen Formel (III)

$R^6$ - $CH_2$ - CO - $R^7$     (III)

in welcher
$R^6$ und $R^7$ die oben angegebenen Bedeutungen haben,
gegebenenfalls in Gegenwart eines Säureakzeptors und gegebenenfalls in Gegenwart eines Verdünnungsmittels umsetzt, oder wenn man

(b) für den Fall, daß in Formel (I)
$R^6$ für Carboxy steht und
$R^7$ für Hydroxy steht sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Alkoxycarbonyl steht und
$R^7$ für Alkoxy steht sowie
Q, $R^1$, $R^2$, $R^3$, $R^4$, $R^5$ und m die oben angegebenen Bedeutungen haben,
mit Wasser, gegebenenfalls in Gegenwart eines Verseifungshilfsmittels und gegebenenfalls in Gegenwart eines Verdünnungsmittels umsetzt, oder wenn man

(c) für den Fall, daß in Formel (I)
$R^6$ für Wasserstoff steht und
$R^7$ für Hydroxy steht sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Carboxy steht und
$R^7$ für Hydroxy steht sowie
Q, $R^1$, $R^2$, $R^3$, $R^4$, $R^5$ und m die oben angegebenen Bedeutungen haben,
pyrolytisch decarboxyliert, oder wenn man

(d) für den Fall, daß in Formel (I)
$R^6$ für Wasserstoff steht und
$R^7$ für Halogen steht sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Wasserstoff steht und
$R^7$ für Hydroxy steht sowie
Q, $R^1$, $R^2$, $R^3$, $R^4$, $R^5$ und m die oben angegebenen Bedeutungen haben,
mit einem Halogenierungsmittel, gegebenenfalls in Gegenwart eines Katalysators und gegebenenfalls in Gegenwart eines organischen Lösungsmittels umsetzt, oder wenn man

(e) für den Fall, daß in Formel (I)
$R^6$ für Wasserstoff steht und
$R^7$ mit Ausnahme von Halogen die oben angegebene Bedeutung hat sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Wasserstoff steht und
$R^7$ für Halogen steht sowie
Q, $R^1$, $R^2$, $R^3$, $R^4$, $R^5$ und m die oben angegebenen Bedeutungen haben,
mit Verbindungen der Formel (IV)

H - $R^7$ (IV)
in welcher
$R^7$ mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder wenn man
(f) für den Fall, daß in Formel (I)
$R^6$ für Wasserstoff steht und
$R^7$ für Alkoxy steht sowie
Q, $R^1$, $R^2$, $R^3$, $R^4$, $R^5$ und m die oben angegebenen Bedeutungen haben,
Verbindungen der allgemeinen Formel (I) in welcher
$R^6$ für Wasserstoff steht und
$R^7$ für Hydroxy steht sowie
Q, $R^1$, $R^2$, $R^3$, $R^4$, $R^5$ und m die oben angegebenen Bedeutungen haben,
mit Alkoholen der Formel (V)
HOR (V)
in welcher
R für Alkyl steht
gegebenenfalls in Gegenwart eines Katalysators umsetzt.

Schließlich wurde gefunden, daß die neuen substituierten Phenoxyphenylpropionsäure-Derivate der Formel (I) hervorragende herbizide Eigenschaften aufweisen.

Überraschenderweise sind die erfindungsgemäßen substituierten Phenoxyphenylpropionsäure-Derivate der Formel (I) gegen Unkräuter wesentlich starker wirksam als 3-(2,4-Dichlor-phenoxy)-6-nitro-benzoesäuremethylester, welches ein strukturell ähnlicher vorbekannter Wirkstoff gleicher Wirkungsrichtung ist.

Halogen bedeutet jeweils Fluor, Chlor, Brom oder lod. Die Kohlenstoffketten in den einzelnen Resten wie beispielsweise Alkyl, Alkenyl, Alkoxy usw. sind jeweils geradkettig oder verzweigt.

Die Erfindung betrifft vorzugsweise Verbindungen der Formel (I), in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Cyano, Carboxy, für gegebenenfalls durch Fluor und/oder Chlor substituiertes $C_1$-$C_4$-Alkyl oder für $C_1$-$C_4$-Alkoxy-carbonyl steht,
$R^7$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxy-carbonyl-$C_1$-$C_2$-alkylamino, Cyanoamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydra zino, $C_1$-$C_4$-Alkylthio, Phenylthio, Benzylthio, $C_1$-$C_4$-Alkoxy-carbonyl-$C_1$-$C_2$-alkylthio oder für die Gruppierung O-$R^8$ steht, worin
$R^8$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl oder für ein Ammonium-, ein $C_1$-$C_4$-Alkylammonium-, ein Natrium-, Kalium-oder Calcium-Äquivalent steht, oder für die Gruppierung

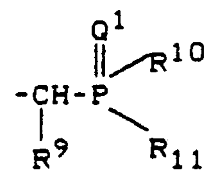

steht, worin
$R^9$ für Wasserstoff, $C_1$-$C_4$-Alkyl, Phenyl, Furyl, Thienyl oder Pyridyl steht,
$R^{10}$ für $C_1$-$C_4$-Alkyl oder $C_1$-$C_4$-Alkoxy steht,

$R^{11}$ für $C_1$-$C_4$-Alkoxy steht und
$Q^1$ für Sauerstoff oder Schwefel steht, oder
$R^8$ für die Gruppierung $-(CH_2)_n-R^{12}$ steht, worin
n für die Zahlen 0, 1 oder 2 steht und
$R^{12}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der Formel (I), in welcher
Q für S oder $SO_2$ steht,
m für die Zahlen 0 oder 1 steht,
$R^1$ für Wasserstoff, Fluor oder Chlor steht,
$R^2$ für Wasserstoff, Fluor oder Chlor steht,
$R^3$ für Trifluormethyl oder Trifluormethylsulfonyl steht,
$R^4$ für Wasserstoff, Fluor oder Chlor steht,
$R^5$ für Wasserstoff, Fluor oder Chlor steht,
$R^6$ für Wasserstoff, Carboxy oder $C_1$-$C_3$-Alkoxy-carbonyl steht,
$R^7$ für Chlor, Hydroxy, Amino, $C_1$-$C_4$-Alkylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxycarbonyl-$C_1$-$C_2$-alkylthio oder für die Gruppierung O-$R^8$ steht, worin
$R^8$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkyl-sulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl oder für ein Ammonium-, ein $C_1$-$C_3$-Alkylammonium-, ein Natrium- oder Kalium-äquivalent steht, oder für die Gruppierung

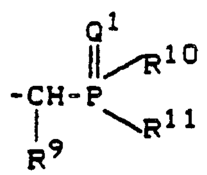

steht, worin
$R^9$ für Wasserstoff, Methyl, Phenyl, Furyl, Thienyl oder Pyridyl steht,
$R^{10}$ für Methoxy oder Ethoxy steht,
$R^{11}$ für Methoxy oder Ethoxy steht und
$Q^1$ für Sauerstoff oder Schwefel steht, oder
$R^8$ für die Gruppierung $-(CH_2)_n-R^{12}$ steht, worin
n für die Zahlen 0, 1 oder 2 steht und
$R^{12}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

Die Erfindung betrifft ganz besonders Verbindungen der Formel (I), in welcher
Q, m, $R^1$ bis $R^6$ die oben angegebene bevorzugten und insbesondere bevorzugten Bedeutungen haben und
$R^7$ für Chlor, Hydroxy, Methoxy, Ethoxy, n- oder iso-Propoxy, n-, iso-, sec- oder tert. Butoxy steht.

Beispiele für die substituierten Phenoxyphenylpropionsäure-Derivate der Formel (I) sind in der nachstehenden Tabelle 1 aufgeführt.

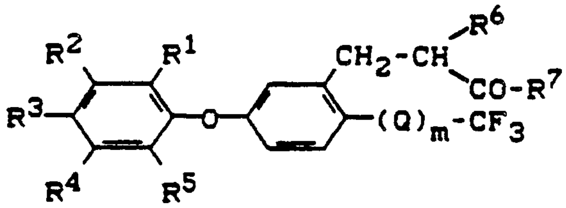

(I)

Tabelle 1: Beispiele für die Verbindungen der Formel (I)

| $R^1$ | $R^2$ | $R^3$ | $R^4$ | $R^5$ | $R^6$ | $R^7$ | Q | m |
|---|---|---|---|---|---|---|---|---|
| Cl | H | $CF_3$ | H | H | H | OH | - | 0 |
| Cl | H | $CF_3$ | H | H | H | OH | S | 1 |
| Cl | H | $CF_3$ | H | H | H | OH | $SO_2$ | 1 |
| Cl | H | $CF_3$ | H | H | H | Cl | - | 0 |
| Cl | H | $CF_3$ | H | H | H | Cl | S | 1 |
| Cl | H | $CF_3$ | H | H | H | $OCH_3$ | - | 0 |
| Cl | H | $CF_3$ | H | H | H | $OC_2H_5$ | - | 0 |
| Cl | H | $CF_3$ | H | H | COOH | OH | - | 0 |
| Cl | H | $CF_3$ | H | H | $COOCH_3$ | $OCH_3$ | - | 0 |
| Cl | H | $CF_3$ | H | H | $COOC_2H_5$ | $OC_2H_5$ | - | 0 |
| Cl | H | $CF_3$ | H | Cl | H | OH | - | 0 |
| Cl | H | $CF_3$ | H | Cl | H | OH | S | 1 |
| Cl | H | $CF_3$ | H | Cl | H | OH | $SO_2$ | 1 |
| Cl | H | $CF_3$ | H | Cl | H | $OCH_3$ | $SO_2$ | 1 |
| Cl | H | $CF_3$ | H | Cl | H | Cl | - | 0 |
| Cl | H | $CF_3$ | H | Cl | H | Cl | S | 1 |
| Cl | H | $CF_3$ | H | Cl | H | $OCH_3$ | - | 0 |
| Cl | H | $CF_3$ | H | Cl | H | $OC_2H_5$ | - | 0 |
| Cl | H | $CF_3$ | H | Cl | COOH | OH | - | 0 |
| Cl | H | $CF_3$ | H | Cl | $COOCH_3$ | $OCH_3$ | - | 0 |
| Cl | H | $CF_3$ | H | Cl | $COOC_2H_5$ | $OC_2H_5$ | - | 0 |
| Cl | H | $CF_3$ | H | F | H | OH | - | 0 |
| Cl | H | $CF_3$ | H | F | H | OH | S | 1 |
| Cl | H | $CF_3$ | H | F | H | OH | $SO_2$ | 1 |
| Cl | H | $CF_3$ | H | F | H | Cl | - | 0 |

Tabelle 1: (Fortsetzung)

| $R^1$ | $R^2$ | $R^3$ | $R^4$ | $R^5$ | $R^6$ | $R^7$ | Q | m |
|---|---|---|---|---|---|---|---|---|
| Cl | H | $CF_3$ | H | F | H | Cl | S | 1 |
| Cl | H | $CF_3$ | H | F | H | $OCH_3$ | - | 0 |
| Cl | H | $CF_3$ | H | F | H | $OC_2H_5$ | - | 0 |
| Cl | H | $CF_3$ | H | F | COOH | OH | - | 0 |
| Cl | H | $CF_3$ | H | F | $COOCH_3$ | $OCH_3$ | - | 0 |
| Cl | H | $CF_3$ | H | F | $COOC_2H_5$ | $OC_2H_5$ | - | 0 |
| Cl | H | $CF_3$ | F | F | H | OH | - | 0 |
| Cl | H | $CF_3$ | F | F | H | $OCH_3$ | - | 0 |
| Cl | H | $CF_3$ | F | F | H | $OC_2H_5$ | - | 0 |
| Cl | H | $CF_3$ | F | F | COOH | OH | - | 0 |
| Cl | H | $CF_3$ | H | Cl | H | $OC_2H_5$ | $SO_2$ | 1 |
| Cl | H | $CF_3$ | F | F | $COOCH_3$ | $OCH_3$ | - | 0 |
| Cl | H | $CF_3$ | F | F | $COOCH_3$ | $OCH_3$ | S | 1 |
| Cl | H | $CF_3$ | Cl | Cl | H | OH | - | 0 |
| Cl | H | $CF_3$ | Cl | Cl | H | Cl | - | 0 |
| Cl | H | $CF_3$ | Cl | Cl | H | $OCH_3$ | - | 0 |
| Cl | H | $CF_3$ | Cl | Cl | H | $OC_2H_5$ | - | 0 |
| Cl | H | $CF_3$ | Cl | Cl | H | OH | S | 1 |
| Cl | H | $CF_3$ | Cl | Cl | H | OH | $SO_2$ | 1 |
| Cl | H | $CF_3$ | Cl | Cl | COOH | OH | - | 0 |
| Cl | H | $CF_3$ | Cl | Cl | $COOH_3$ | $OCH_3$ | - | 0 |
| Cl | H | $CF_3$ | Cl | Cl | $COOC_2H_5$ | $OC_2H_5$ | - | 0 |
| Cl | H | $CF_3$ | Cl | Cl | $COOC_2H_5$ | $OC_2H_5$ | S | 1 |
| Cl | H | $CF_3$ | F | Cl | H | OH | - | 0 |
| Cl | H | $CF_3$ | F | Cl | H | Cl | - | 0 |
| Cl | H | $CF_3$ | F | Cl | H | OH | S | 1 |

Tabelle 1: (Fortsetzung)

| $R^1$ | $R^2$ | $R^3$ | $R^4$ | $R^5$ | $R^6$ | $R^7$ | Q | m |
|---|---|---|---|---|---|---|---|---|
| Cl | H | $CF_3$ | F | Cl | H | OH | $SO_2$ | 1 |
| Cl | H | $CF_3$ | F | Cl | H | $OCH_3$ | - | 0 |
| Cl | H | $CF_3$ | F | Cl | H | $OC_2H_5$ | - | 0 |
| Cl | H | $CF_3$ | F | Cl | COOH | OH | - | 0 |
| Cl | H | $CF_3$ | F | Cl | $COOCH_3$ | $OCH_3$ | - | 0 |
| Cl | H | $CF_3$ | F | Cl | $COOC_2H_5$ | $OC_2H_5$ | - | 0 |
| Cl | H | $CF_3$ | H | F | H | $OCH_3$ | $SO_2$ | 1 |
| Cl | H | $CF_3$ | H | Cl | H | $OC_3H_7$ | - | 0 |
| Cl | H | $CF_3$ | H | Cl | H | $OCH(CH_3)_2$ | - | 0 |
| Cl | H | $CF_3$ | H | Cl | H | $OC_4H_9$ | - | 0 |
| Cl | H | $CF_3$ | H | Cl | H | $OCH_2CH(CH_3)_2$ | - | 0 |
| Cl | H | $CF_3$ | H | Cl | H | $OCH_2COOCH_3$ | - | 0 |
| Cl | H | $CF_3$ | H | Cl | H | $SCH_2COOCH_3$ | - | 0 |
| Cl | H | $CF_3$ | H | Cl | H | $OCH_2CH_2OCH_3$ | - | 0 |
| Cl | H | $CF_3$ | H | Cl | H | $OCH_2CH_2SC_2H_5$ | - | 0 |
| Cl | H | $CF_3$ | H | Cl | H | $SC_2H_5$ | - | 0 |
| Cl | H | $CF_3$ | H | Cl | H | $SC_4H_9$ | - | 0 |
| Cl | H | $CF_3$ | H | Cl | H | $OCH_2P(=O)(OC_2H_5)_2$ | - | 0 |
| Cl | H | $CF_3$ | H | Cl | H | $NHCH(CH_3)_2$ | - | 0 |
| Cl | H | $CF_3$ | H | Cl | H | $N(CH_3)_2$ | - | 0 |
| Cl | H | $CF_3$ | H | Cl | H | $NHSO_2CH_3$ | - | 0 |
| Cl | H | $CF_3$ | H | Cl | H | $NHOC_2H_5$ | - | 0 |
| Cl | H | $CF_3$ | H | Cl | H | $NHCH_2COOC_2H_5$ | - | 0 |

Tabelle 1: (Fortsetzung)

| $R^1$ | $R^2$ | $R^3$ | $R^4$ | $R^5$ | $R^6$ | $R^7$ | Q | m |
|---|---|---|---|---|---|---|---|---|
| Cl | H | $CF_3$ | H | Cl | H | 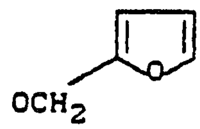 | - | 0 |
| Cl | H | $CF_3$ | H | Cl | H | 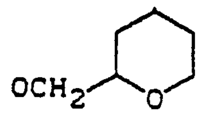 | - | 0 |
| Cl | H | $CF_3$ | H | F | H | $OC_2H_5$ | $SO_2$ | 1 |

Verwendet man für das erfindungsgemäße Verfahren (a) beispielsweise 2-Trifluormethyl-5-(2-Chlor-4-trifluormethyl-phenoxy)-benzylchlorid und Malonsäure-diethylester als Ausgangsstoffe, so kann der Reaktionsablauf durch das folgende Formelschema wiedergegeben werden:

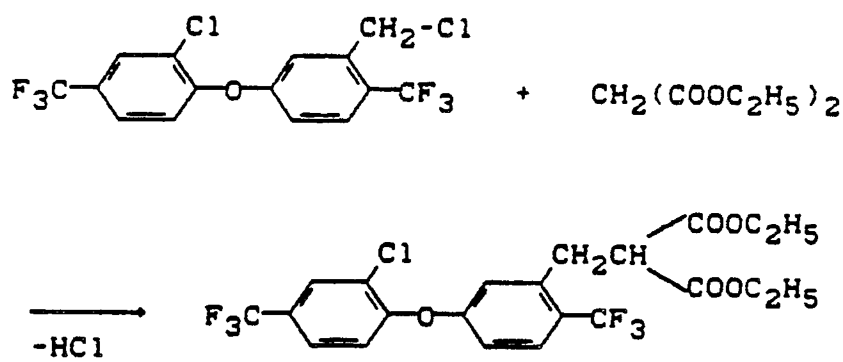


Verwendet man für das erfindungsgemäße Verfahren (b) beispielsweise α-[2-Trifluormethylthio-5-(2-chlor-6- fluor-4-trifluormethyl-phenoxy)-benzyl]-malonsäure-bis-methylester als Ausgangsstoff, so kann der Reaktionsablauf durch das folgende Formelschema wiedergegeben werden:

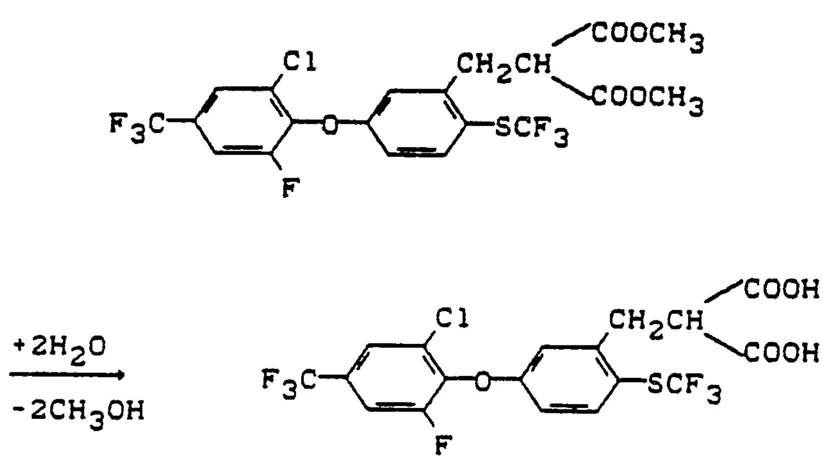

Verwendet man für das erfindungsgemäße Verfahren (c) beispielsweise α-[2-Trifluormethyl-5-(2,3,6-trichlor-4-trifluormethyl-phenoxy)-benzyl]-malonsäure als Ausgangsstoff, so kann der Reaktionsablauf durch das folgende Formelschema skizziert werden:

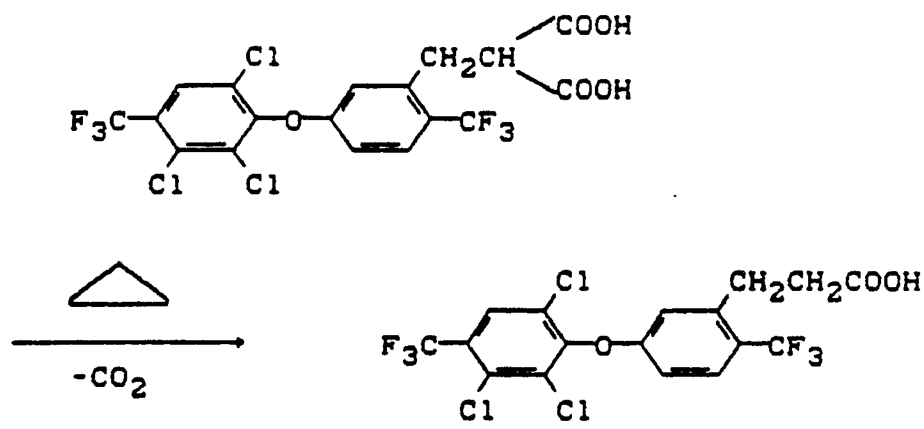


Verwendet man für das erfindungsgemäße Verfahren (d) beispielsweise β-[2-Trifluormethylsulfonyl-5-(2,6-dichlor-4-trifluormethyl-phenoxy)-phenyl]-propionsäure und Thionylchlorid als Ausgangsstoffe, so kann der Reaktionsablauf durch das folgende Formelschema skizziert werden:

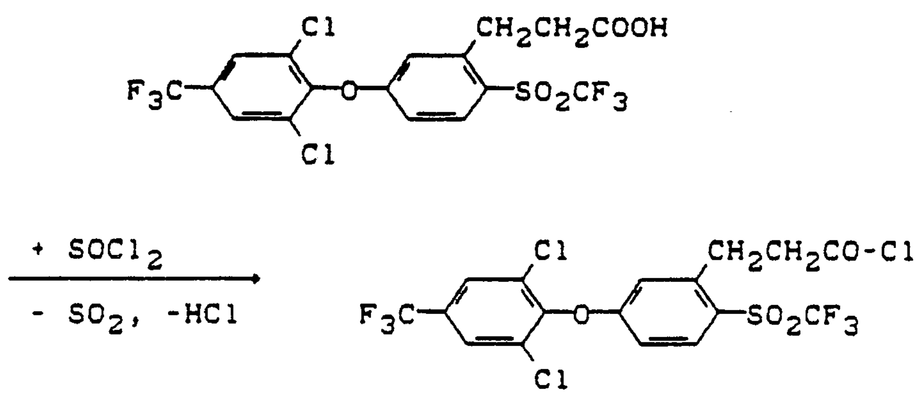


Verwendet man für das erfindungsgemäße Verfahren (e) beispielsweise β-[2-Trifluormethylthio-5-(2,3,6-trifluor-4-trifluormethyl-phenoxy)-phenyl]-propionsäurechlorid und Mercaptoessigsäuremethylester als Ausgangsstoffe, so kann der Reaktionsablauf durch das folgende Formelschema skizziert werden:

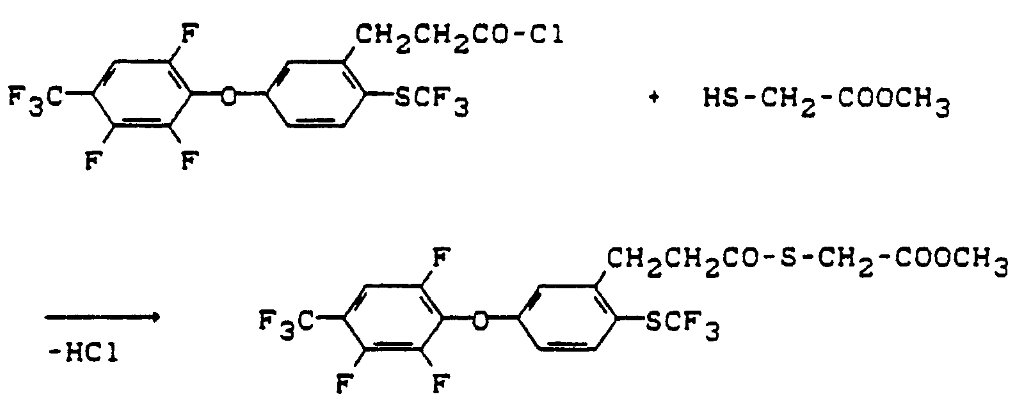


Verwendet man für das erfindungsgemäße Verfahren (f) beispielsweise β-[2-Trifluormethyl-5-(2,6-dichlor-3-fluor-4-trifluormethyl-phenoxy)-phenyl]propionsäure und Butanol als Ausgangsstoffe, so kann der Reaktionsablauf durch das folgende Formelschema skizziert werden:

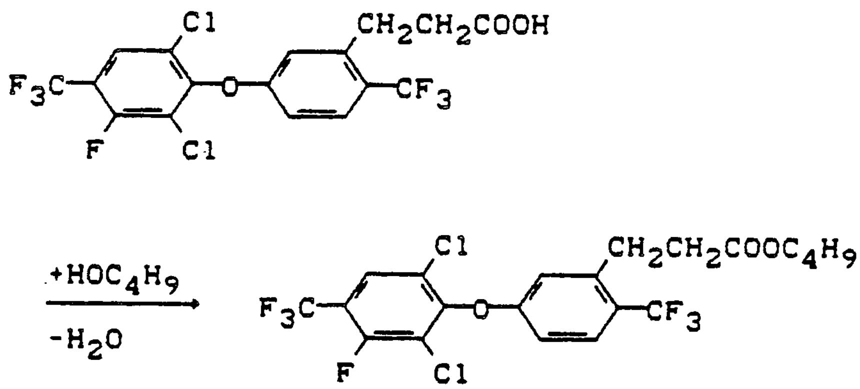


Die beim erfindungsgemäßen Verfahren (a) zur Herstellung von Verbindungen der Formel (I) als Ausgangsstoffe zu verwendenden substituierten Phenoxybenzylhalogenide sind durch die Formel (II) allgemein definiert.

In Formel (II) haben Q, $R^1$, $R^2$, $R^3$, $R^4$, $R^5$ und m vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden und X steht vorzugsweise für Chlor oder Brom.

Beispiele für die Ausgangsstoffe der Formel (II) sind in der nachstehenden Tabelle 2 aufgeführt.

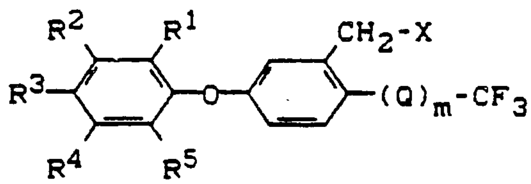

(II)

Tabelle 2: Beispiele für die Verbindungen der Formel (II)

| $R^1$ | $R^2$ | $R^3$ | $R^4$ | $R^5$ | Q | m | X |
|---|---|---|---|---|---|---|---|
| Cl | H | $CF_3$ | H | H | - | 0 | Cl |
| Cl | H | $CF_3$ | H | H | - | 0 | Br |
| Cl | H | $CF_3$ | H | H | S | 1 | Br |
| Cl | H | $CF_3$ | H | H | $SO_2$ | 1 | Cl |
| Cl | H | $CF_3$ | H | Cl | - | 0 | Cl |
| Cl | H | $CF_3$ | H | Cl | - | 0 | Br |
| Cl | H | $CF_3$ | H | Cl | S | 1 | Br |
| Cl | H | $CF_3$ | H | Cl | $SO_2$ | 1 | Cl |

Tabelle 2: (Fortsetzung)

| $R^1$ | $R^2$ | $R^3$ | $R^4$ | $R^5$ | Q | m | X |
|---|---|---|---|---|---|---|---|
| Cl | H | $CF_3$ | H | F | - | 0 | Cl |
| Cl | H | $CF_3$ | H | F | - | 0 | Br |
| Cl | H | $CF_3$ | H | F | S | 1 | Br |
| Cl | H | $CF_3$ | H | F | $SO_2$ | 1 | Cl |
| Cl | H | $CF_3$ | F | F | - | 0 | Cl |
| Cl | H | $CF_3$ | F | F | - | 0 | Br |
| Cl | H | $CF_3$ | F | F | S | 1 | Br |
| Cl | H | $CF_3$ | F | F | $SO_2$ | 1 | Cl |
| Cl | H | $CF_3$ | Cl | Cl | - | 0 | Cl |
| Cl | H | $CF_3$ | Cl | Cl | - | 0 | Br |
| Cl | H | $CF_3$ | Cl | Cl | S | 1 | Br |
| Cl | H | $CF_3$ | Cl | Cl | $SO_2$ | 1 | Cl |
| Cl | H | $CF_3$ | F | Cl | - | 0 | Cl |
| Cl | H | $CF_3$ | F | Cl | - | 0 | Br |
| Cl | H | $CF_3$ | F | Cl | S | 1 | Br |
| Cl | H | $CF_3$ | F | Cl | $SO_2$ | 1 | Cl |
| Cl | H | $SO_2CF_3$ | H | H | - | 0 | Br |
| Cl | H | $SO_2CF_3$ | H | Cl | - | 0 | Br |
| Cl | H | $CF_3$ | H | Cl | $SO_2$ | 1 | Br |

Die Ausgangsstoffe der Formel (II) sind noch nicht aus der Literatur bekannt. Man erhält die neuen substituierten Phenoxybenzylhalogenide der Formel (II), wenn man substituierte Phenoxytoluole der allgemeinen Formel (VI)

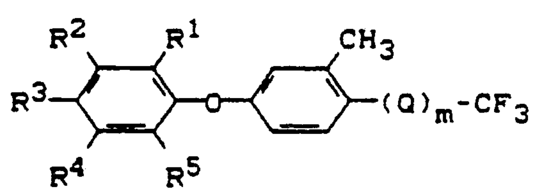

(VI)

in welcher
Q, $R^1$, $R^2$, $R^3$, $R^4$, $R^5$ und m die oben angegebenen Bedeutungen haben,
mit Halogenierungsmitteln, wie z. B. Chlor, Brom, Sulfurylchlorid, N-Chlor- oder N-Brom-succinimid, gegebenenfalls unter Belichtung, beispielsweise mit Quecksilbertauchlampen, gegebenenfalls in Gegenwart von Katalysatoren, wie z. B. Benzoylperoxid oder Azo-bis-isobutyronitril, und gegebenenfalls in Gegenwart von Verdünnungsmitteln, wie z. B. Tetrachlormethan, bei Temperaturen zwischen 20 °C und 150 °C umsetzt.

Die als Zwischenprodukte benötigten substituierten Phenoxytoluole sind durch die Formel (VI) allgemein definiert. In Formel (VI) haben Q, $R^1$, $R^2$, $R^3$, $R^4$, $R^5$ und m vorzugsweise bzw. insbesondere

diejenigen Bedeutungen, die bereits im Zusammenhang mit der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden.

Beispiele für die Zwischenprodukte der Formel (VI) sind in der nachstehenden Tabelle 3 aufgeführt.

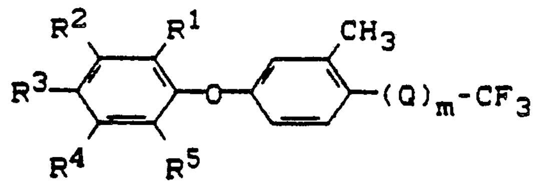

(VI)

Tabelle 3

| Beispiele für die Zwischenprodukte der Formel (VI) | | | | | | |
|---|---|---|---|---|---|---|
| $R^1$ | $R^2$ | $R^3$ | $R^4$ | $R^5$ | Q | m |
| Cl | H | $CF_3$ | H | H | - | 0 |
| Cl | H | $CF_3$ | H | H | S | 1 |
| Cl | H | $CF_3$ | H | H | $SO_2$ | 1 |
| Cl | H | $CF_3$ | H | Cl | - | 0 |
| Cl | H | $CF_3$ | H | Cl | S | 1 |
| Cl | H | $CF_3$ | H | Cl | $SO_2$ | 1 |
| Cl | H | $CF_3$ | H | F | - | 0 |
| Cl | H | $CF_3$ | H | F | S | 1 |
| Cl | H | $CF_3$ | H | F | $SO_2$ | 1 |
| Cl | H | $CF_3$ | F | F | - | 0 |
| Cl | H | $CF_3$ | F | F | S | 1 |
| Cl | H | $CF_3$ | F | F | $SO_2$ | 1 |
| Cl | H | $CF_3$ | Cl | Cl | - | 0 |
| Cl | H | $CF_3$ | Cl | Cl | S | 1 |
| Cl | H | $CF_3$ | Cl | Cl | $SO_2$ | 1 |
| Cl | H | $CF_3$ | F | Cl | - | 0 |
| Cl | H | $CF_3$ | F | Cl | S | 1 |
| Cl | H | $CF_3$ | F | Cl | $SO_2$ | 1 |
| Cl | H | $SO_2CF_3$ | H | H | - | 0 |
| Cl | H | $SO_2CF_3$ | H | Cl | - | 0 |

Die Zwischenprodukte der Formel (VI) sind noch nicht aus der Literatur bekannt. Man erhält die neuen substituierten Phenoxytoluole der Formel (VI), wenn man entsprechende Halogen-benzol-Derivate der allgemeinen Formel (VII)

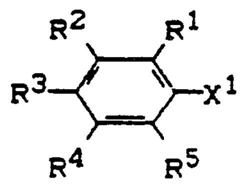

(VII)

in welcher
$R^1$, $R^2$, $R^3$, $R^4$ und $R^5$ die oben angegebenen Bedeutungen haben und
$X^1$ für Fluor oder Chlor steht,

mit Phenolderivaten der allgemeinen Formel (VIII)

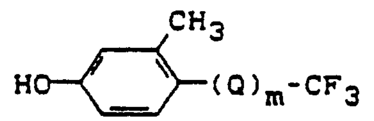

(VIII)

in welcher
Q und m die oben angegebenen Bedeutungen haben
in Gegenwart eines Säureakzeptors, wie z. B. Natrium-oder Kalium-hydroxid, und in Gegenwart eines Verdün nungsmittels, wie z. B. Dimethylsulfoxid, bei Temperaturen zwischen 20 °C und 150 °C umsetzt und nach üblichen Methoden aufarbeitet.

Die als Zwischenprodukte zu verwendenden Halogenbenzol-Derivate sind durch die Formel (VII) allgemein definiert. In Formel (VII) haben $R^1$, $R^2$, $R^3$, $R^4$ und $R^5$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und $X^1$ steht vorzugsweise für Chlor oder Fluor.

Als Beispiele für die Zwischenprodukte der Formel (VII) seien genannt:
3,4-Dichlor-benzotrifluorid, 3,4,5-Trichlor-benzotrifluorid, 3,4-Dichlor-5-fluor-benzotrifluorid, 2,3,4,5-Tetrachlor-benzotrifluorid, 3,5-Dichlor-2,4-difluor-benzotrifluorid und 3-Chlor-4,5-difluor-benzotrifluorid.

Die Verbindungen der Formel (VII) sind bekannt und/oder können nach an sich bekannten Verfahren hergestellt werden (vgl. J. Chem. Soc. 1969, 213-217; FR-A 2 538 380 (Chem. Abstracts 102 (1985), 61914x); EP-A 180 057; US-P 4 388 472 und vgl. Herstellungsbeispiele).

Die weiter als Zwischenprodukte benötigten Phenolderivate sind durch die Formel (VIII) allgemein definiert.

In Formel (VIII) haben Q und m vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt angegeben wurden.

Als Beispiele für die Zwischenprodukte der Formel (VIII) seien genannt:
3-Methyl-4-trifluormethyl-phenol, 3-Methyl-4-trifluormethylthio-phenol und 3-Methyl-4-trifluormethylsulfonylphenol.

Die Verbindungen der Formel (VIII) sind bekannt und/oder können nach an sich bekannten Verfahren hergestellt werden (vergl. EP-A 206 951, DE-A 1 257 784).

Für den Fall, daß in Formel (VI) m für Null steht, erhält man die Verbindungen der Formel (VI) auch, wenn man Phenoxytoluole der allgemeinen Formel (IX)

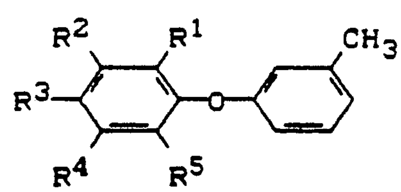

(IX)

in welcher
$R^1$, $R^2$, $R^3$, $R^4$ und $R^5$ die oben angegebenen Bedeutungen haben,
mit Hydrogenfluorid/Tetrachlormethan bei Temperaturen zwischen 0 °C und 150 °C und Drücken zwischen 1 und 50 bar umsetzt und nach üblichen Methoden aufarbeitet.

Die als Zwischenprodukte zu verwendenden Phenoxytoluole sind durch die Formel (IX) allgemein definiert.

In Formel (IX) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Als Beispiele für die Zwischenprodukte der Formel (IX) seien genannt:
3-(2-Chlor-4-trifluormethyl-phenoxy)-toluol, 3-(2,6-Dichlor-4-trifluormethyl-phenoxy)-toluol, 3-(2-Chlor-6-fluor-4-trifluormethyl-phenoxy)-toluol, 3-(2,3,6-Trichlor-4-trifluormethyl-phenoxy)-toluol, 3-(2,6-Dichlor-3-fluor-4-trifluormethyl-phenoxy)-toluol und 3-(2,3,6-Trifluor-4-trifluormethyl-phenoxy)-toluol.

Die Verbindungen der Formel (IX) sind bekannt und/oder können nach an sich bekannten Verfahren hergestellt werden (vergl. DE-OS 2 333 848, DE-OS 2 520 815).

Für den Fall, daß in Formel (VI) m für 3 und Q für SO oder $SO_2$ steht, erhält man die entsprechenden Verbin dungen der Formel (VI) auch, wenn man die wie oben beschrieben erhältlichen Verbindungen der Formel (VI), in welcher m für 1 und Q für S steht, mit Oxidationsmitteln, wie z. B. Hydrogenperoxid, in Gegenwart von Wasser und gegebenenfalls in Gegenwart eines organischen Lösungsmittels, wie z. B. Essigsäure, bei Temperaturen zwischen 0° C und 100° C umsetzt und nach üblichen Methoden aufarbeitet.

Die beim erfindungsgemäßen Verfahren (a) zur Herstellung von Verbindungen der Formel (I) weiter als Ausgangsstoffe zu verwendenden Carbonylverbindungen sind durch die Formel (III) allgemein definiert.

In Formel (III) haben $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^6$ und $R^7$ angegeben wurden.

Als Beispiele für die Ausgangsstoffe der Formel (III) seien genannt:
Malonsäure sowie Malonsäure-dimethylester, -diethylester, -dipropylester und -dibutylester.

Die Ausgangsstoffe der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid, ferner auch Alkohole wie Methanol, Ethanol, Propanol, Isopropanol, Butanol, Isobutanol, sec-Butanol und tert.-Butanol.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylben zylamin, Pyridin, 1,5-Diazabicyclo-[4,3,0]-non-5-en (DBN), 1,8-Diazabicyclo[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem großeren Bereich variiert werden. Im allgemeinen arbeitet man bei Temperaturen zwischen 0° C und 150° C, vorzugsweise bei Temperaturen zwischen 10° C und 120° C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) als Ausgangsstoffe zu verwendenden Verbindungen sind durch die For mel (I) mit der Maßgabe, daß $R^6$ für Alkoxycarbonyl steht und $R^7$ für Alkoxy steht, allgemein definiert.

In diesem Fall haben Q, $R^1$, $R^2$, $R^3$, $R^4$, $R^5$ und m vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden;
$R^6$ steht vorzugsweise für $C_1$-$C_4$-Alkoxy-carbonyl, insbesondere für $C_1$-$C_3$-Alkoxy-carbonyl, und
$R^7$ steht vorzugsweise für $C_1$-$C_4$-Alkoxy, insbesondere für $C_1$-$C_3$-Alkoxy.

Die oben beschriebenen Ausgangsstoffe der Formel (I) für Verfahren (b) sind erfindungsgemäße, neue Verbindungen; sie können nach dem erfindungsgemäßen Verfahren (a) hergestellt werden.

Verfahren (b) wird vorzugsweise in Gegenwart eines Verseifungshilfsmittels durchgeführt. Als solche kommen insbesondere starke Säuren, wie z. B. Salzsäure oder Schwefelsäure, oder Alkalihydroxide, wie z. B. Natriumhydroxid oder Kaliumhydroxid, in Betracht.

Verfahren (b) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol, eingesetzt.

Die Reaktionstemperaturen können bei dem erfindungsgemäßen Verfahren (b) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung von Verfahren (b) werden je Mol Ausgangsverbindung der Formel (I) im allgemeinen zwischen 0,1 und 10 Mol, vorzugsweise zwischen 0,5 und 5 Mol, Verseifungshilfsmittel eingesetzt. Die Reaktionskomponenten werden im allgemeinen bei Raumtemperatur zusammen gegeben und das Reaktionsgemisch wird, gegebenenfalls bei erhöhter Temperatur, bis zum Reaktionsende gerührt. Das gegebenenfalls nach Einengen, Abkühlen und Ansäuern kristallin anfallende Reaktionsprodukt kann durch Absaugen isoliert werden.

Die beim erfindungsgemäßen Verfahren (c) als Ausgangsstoffe zu verwendenden Verbindungen sind durch die Formel (I) mit der Maßgabe, daß $R^6$ für Carboxy steht und $R^7$ für Hydroxy steht, allgemein definiert.

In diesem Fall haben Q, $R^1$, $R^2$, $R^3$, $R^4$, $R^5$ und m vorzugsweise bzw. insbesondere diejenigen Bedeutungen, die bereits oben im Rahmen der Beschreibung der erfindungsgemäßen Verbindungen der Formel (I) vorzugsweise bzw als insbesondere bevorzugt für Q, $R^1$, $R^2$, $R^3$, $R^4$, $R^5$ und m angegeben wurden.

Die oben beschriebenen Ausgangsstoffe der Formel (I) für Verfahren (c) sind erfindungsgemäße, neue Verbindungen; sie können nach den erfindungsgemäßen Verfahren (a) und (b) hergestellt werden.

Das erfindungsgemäße Verfahren (c) wird als pyrolytische Decarboxylierung durchgeführt. Hierzu werden die Ausgangsstoffe der Formel (I) "in Substanz", d. h. ohne weitere Zusätze auf Temperaturen zwischen 100 °C und 300 °C, vorzugsweise zwischen 150 °C und 200 °C, erhitzt, bis die Gasentwicklung abgeklungen ist.

Die Produkte dar Decarboxylierung fallen nach dem Abkühlen in kristalliner Form an.

Die beim erfindungsgemäßen Verfahren (d) als Ausgangsstoffe zu verwendenden Verbindungen sind durch die Formel (I) mit der Maßgabe, daß $R^6$ für Wasserstoff steht und $R^7$ für Hydroxy steht, allgemein definiert.

In diesem Fall haben Q, $R^1$, $R^2$, $R^3$, $R^4$, $R^5$ und m vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden.

Die oben beschriebenen Ausgangsstoffe der Formel (I) für Verfahren (d) sind erfindungsgemäße, neue Verbindungen; sie können nach dem erfindungsgemäßen Verfahren (c) hergestellt werden.

Verfahren (d) wird unter Verwendung eines Halogenierungsmittels durchgeführt. Es können die üblichen Mittel für die Umsetzung von Carbonsäuren zu Carbonsäurehalogeniden eingesetzt werden. Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (d) wird gegebenenfalls in Gegenwart eines Katalysators durchgeführt. Es können die für die Herstellung von Säurechloriden aus Säuren üblichen Katalysatoren, wie z. B. Pyridin oder Dimethylformamid, verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem großeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden

Die beim erfindungsgemäßen Verfahren (e) als Ausgangsstoffe zu verwendenden Verbindungen sind durch die Formel (I) mit der Maßgabe, daß $R^6$ für Wasserstoff steht und $R^7$ für Halogen steht, allgemein definiert.

In diesem Fall haben Q, $R^1$, $R^2$, $R^3$, $R^4$, $R^5$ und m vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden und $R^7$ steht vorzugsweise für Fluor, Chlor oder Brom, insbesondere für Chlor.

Die oben beschriebenen Ausgangsstoffe der Formel (I) für Verfahren (e) sind erfindungsgemäße, neue Verbindungen; sie können nach dem erfindungsgemäßen Verfahren (d) hergestellt werden.

Bei den als weitere Ausgangsstoffe für Verfahren (e) einzusetzenden Verbindungen der Formel (IV) hat $R^7$ vorzugsweise bzw. insbesondere diejenige Bedeutung, die bereits oben im Rahmen der Beschreibung der erfindungsgemäßen Verbindungen der Formel (I) vorzugsweise bzw. als insbesondere bevorzugt für $R^7$ angegeben wurde, wobei Halogen ausgenommen ist.

Als Beispiele für diese Ausgangsstoffe zu Verfahren (e) seien genannt:

Methylamin, Ethylamin, Propylamin, Isopropylamin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxyethanol, 2-Benzylthio-ethanol, Hydroxymethanphosphonsäure-diethylester und -dimethylester, 1-Hydroxy-ethanphosphonsäure-dimethylester und -diethylester, 1-Hydroxy -1-phenyl-methanphosphonsäure-dimethylester und -diethylester, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Das erfindungsgemäße Verfahren (e) wird vorzugsweise unter Verwendung von Verdünnungsmitteln durchgeführt. Als Verdünnungsmittel kommen dabei praktisch alle inerten organischen Lösungsmittel in Frage. Hierzu gehoren vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (e) alle üblicherweise für derartige Umsetzungen verwendbaren Säurebindemittel eingesetzt werden Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN) 1,8-Diazabicyc lo-[5,4,0]-undec-7-en (DBU) und 1,4-Diazabicyclo-[2,2,2]-octan (DABCO).

Die Reaktionstemperaturen können bei dem erfindungsgemäßen Verfahren (e) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können..

Die beim erfindungsgemäßen Verfahren (f) als Ausgangsstoffe zu verwendenden Verbindungen sind

durch die Formel (I) mit der Maßgabe, daß $R^6$ für Wasserstoff steht und $R^7$ für Hydroxy steht, allgemein definiert.

In diesem Fall haben Q, $R^1$, $R^2$, $R^3$, $R^4$, $R^5$ und m vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für Q, $R^1$, $R^2$, $R^3$, $R^4$, $R^5$ und m angegeben wurden.

Die oben beschriebenen Ausgangsstoffe der Formel (I) für Verfahren (f) sind erfindungsgemäße, neue Verbindungen; sie können nach dem erfindungsgemäßen Verfahren (c) hergestellt werden.

Die bei Verfahren (f) weiter als Ausgangsstoffe einzusetzenden Alkohole sind durch die Formel (V) allgemein definiert. In Formel (V) steht R vorzugsweise für $C_1$-$C_6$-Alkyl, insbesondere für $C_1$-$C_4$-Alkyl.

Als Beispiele für die Ausgangsstoffe der Formel (V) seien genannt:

Methanol, Ethanol, Propanol, Isopropanol, Butanol, Isobutanol, sec-Butanol und tert.-Butanol.

Verfahren (f) wird gegebenenfalls in Gegenwart eines Katalysators durchgeführt. Als solche kommen vorzugsweise starke Säuren, wie z. B. Schwefelsäure, Methansulfonsäure oder p-Toluolsulfonsäure in Betracht.

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (f) wird der Alkohol der Formel (V) vorzugsweise in einem so großen Überschuß eingesetzt, daß er auch als Verdünnungsmittel dient. Die Reaktionskomponenten werden im allgemeinen bei Raumtemperatur vermischt und dann vorzugsweise bei erhöhter Temperatur bis zum Ende der Umsetzung gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natür liche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage; ferner auch
2,4-Dichlorphenoxyessigsäure (2,4-D);
4-(2,4-Dichlorphenoxy)-buttersäure (2,4-DB); 2,4-Dichlorphenoxypropionsäure (2,4-DP);
5-(2-Chlor-4-trifluormethyl-phenoxy)-2-nitro-benzoesäure (ACIFLUORFEN); Chloressigsäure-N-(methoxymethyl)2,6-diethylanilid (ALACHLOR);
2-Chlor-4-ethylamino-6-isopropylamino-1,3,5-triazin (ATRAZIN);
3-Isopropyl-2,1,3-benzothiadiazin-4-on-2,2-dioxid (BENTAZON);
Methyl-5-(2,4-dichlorphenoxy)-2-nitrobenzoat (BIFENOX);
3,5-Dibrom-4-hydroxy-benzonitril (BROMOXYNIL);
Ethyl-2-{[(4-chlor-6-methoxy-2-pyrimidinyl)-aminocarbonyl]-aminosulfonyl}-benzoat (CHLORIMURON);
2-chlor-N-{[C(4-methoxy-6-methyl-1,3,5-triazin-2-yl)-amino]-carbonyl}-benzolsulfonamid (CHLORSULFURON);
N,N-Dimethyl-N′-(3-chlor-4-methylphenyl)-harnstoff (CHLORTOLURON);
exo-1-Methyl-4-(1-methylethyl)-2-(2-methylphenyl-methoxy)-7-oxabicyclo-(2,2,1)-heptan (CINMETHYLIN);
3,6-Dichlor-2-pyridincarbonsäure (CLOPYRALID);
2-Chlor-4-ethylamino-6-(3-cyanopropylamino)-1,3,5-triazin (CYANAZIN);
2-[4-(2,4-Dichlorphenoxy)-phenoxy]-propionsäure, deren Methyl-oder deren Ethylester (DICLOFOP);
2-[(2-Chlorphenyl)-methyl]-4,4-dimethylisoxazolidin-3-on (DIMETHAZONE);
N,N-Di-n-propyl-thiocarbamidsäure-S-ethylester (EPTAME);
4-Amino-6-t-butyl-3-ethylthio-1,2,4-triazin-5(4H)-on (ETHIOZIN);
2-{4-[(6-Chlor-2-benzoxazolyl)-oxy]-phenoxy}-propansäure, deren Methyl- oder deren Ethylester (FENOXAPROP);
2-[4-(5-Trifluormethyl-2-pyridyloxy)-phenoxy]-propansäure oder deren Butylester (FLUAZIFOP);

[(4-Amino-3,5-dichlor-6-fluor-2-pyridinyl)-oxy]-essigsäure bzw. deren 1-Methylheptylester (FLUROXYPYR);
5-(2-Chlor-4-trifluormethyl-phenoxy)-N-methylsulfonyl-2-nitrobenzamid (FOMESAFEN);
N-Phosphonomethyl-glycin (GLYPHOSATE);
2-{4-[(3-Chlor-5-(trifluormethyl)-2-pyridinyl)-oxy]-phenoxy}-propansäure bzw. deren Ethylester (HALOXYFOP);
3-Cyclohexyl-6-dimethylamino-1-methyl-1,3,5-triazin-2,4-dion (HEXAZINONE);
Methyl-2-[4,5-dihydro-4-methyl-4-(1-methylethyl)-5-oxo-1H-imidazol-2-yl]-4(5)-methylbenzoat (IMAZAMETHABENZ);
2-(4,5-Dihydro-4-methyl-4-isopropyl-5-oxo-1H-imidazol-2-yl)-pyridin-3-carbonsäure (IMAZAPYR);
2-[5-Methyl-5-(1-methylethyl)-4-oxo-2-imidazolin-2-yl]-3-chinolincarbonsäure (IMAZAQUIN);
2-[4,5-Dihydro-4-methyl-4-isopropyl-5-oxo-(1H)-imidazol-2-yl]-5-ethyl-pyridin-3-carbonsäure (IMAZETHAPYR);
3,5-Diiod-4-hydroxybenzonitril (IOXYNIL);
N,N-Dimethyl-N′-(4-isopropylphenyl)-harnstoff (ISOPROTURON);
(2-Ethoxy-1-methyl-2-oxo-ethyl)-5-[2-chlor-4-(trifluormethyl)-phenoxy]-2-nitrobenzoat (LACTOFEN);
(2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET);
2-Ethyl-6-methyl-N-(1-methyl-2-methoxyethyl )-chloracetanilid (METOLACHLOR);
2-{[[((4-Methoxy-6-methyl-1,3,5-triazin-2-yl)-amino)carbonyl]-amino]-sulfonyl}-benzoesäure oder deren Methylester (METSULFURON);
1-(3-Trifluormethyl-phenyl)-4-methylamino-5-chlor-6-pyridazon (NORFLURAZON);
4-(Di-n-propylamino)-3,5-dinitrobenzolsulfonamid (ORYZALIN);
(2-Chlor-4-trifluormethylphenyl)-(3-ethoxy-4-nitro-phenyl)-ether (OXYFLUORFEN);
N-(1-Ethylpropyl)-3,4-dimethyl-2,6-dinitroanilin (PENDIMETHALIN);
0-(6-Chlor-3-phenyl-pyridazin-4-yl)-S-octyl-thiocarbonat (PYRIDATE);
2-[1-(Ethoxamino)-butyliden]-5-(2-ethylthiopropyl)-1,3-cyclohexadion (SETHOXYDIM);
2-Chlor-4,6-bis-(ethylamino)-1,3,5-triazin (SIMAZIN);
4-Ethylamino-2-t-butylamino-6-methylthio-s-triazin (TERBUTRYNE);
3-[[[[(4-Meth-oxy-6-me-thyl-1,3,5-tri-azin-2-yl)-amino]-carbonyl]-amino]-sulfonyl]-thiophen-2-carbonsäuremethylester (THIAMETURON);
N,N-Diisopropyl-S-(2,3,3-trichlorallyl)-thiolcarbamat (TRIALLATE);
2,6-Dinitro-4-trifluormethyl-N,N-dipropylanilin (TRIFLURALIN);
2-[4-(6-Chlor-chinoxalin-2-yl-oxy)-phenoxy]-propionsäure-ethylester (QUIZALOFOPETHYL);

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen be reiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,005 und 5 kg Wirkstoff pro Hektar Bodenfläche, vorzugsweise zwischen 0,01 und 3 kg pro ha.

Die Herstellung und die Verwendung der erfindungsgemäßen Wirkstoffe geht aus den nachfolgenden Beispielen hervor.

## Herstellungsbeispiele

### Beispiel 1

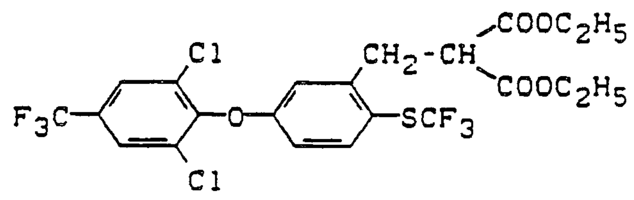

[Verfahren (a)]

Eine durch Verrühren von, 4,0 g (0,025 Mol) Malonsäurediethylester, 1,4 g Natriummethylat und 25 ml Ethanol hergestellte Lösung wird zur Trockne eingedampft. Von dem so erhaltenen Natriumsalz von Malonsäurediethylester werden 3,7 g (0,020 Mol) entnommen und zusammen mit 8,0 g (0,016 Mol) 2-Trifluormethylthio-5-(2,6-dichlor-4-trifluormethyl-phenoxy)-benzylbromid und 70 ml Acetonitril 20 Stunden unter Rückfluß zum Sieden erhitzt. Anschließend wird eingeengt, der Rückstand mit einer Mischung aus Kieselgel und Natriumsulfat verrührt und filtriert. Das Filtrat wird eingeengt, der Rückstand mit einigen ml Hexan verrührt und das kristalline Produkt durch Absaugen isoliert.

Man erhält 1,4 g (16 % der Theorie) α-[2-Trifluormethylthio-5-(2,6-dichlor-4-trifluormethyl-phenoxy)-benzyl]-malonsäure-bis-ethylester vom Schmelzpunkt 83° C.

Beispiel 2

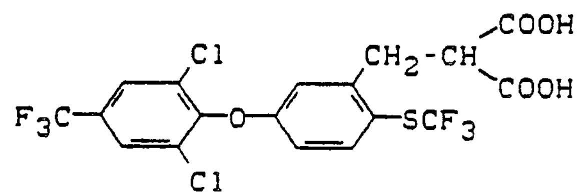

[Verfahren (b)]

Eine Mischung aus 2,6 g (5 m Mol) α-[2-Trifluormethylthio-5- (2,6-dichlor-4-trifluormethyl-phenoxy)-benzyl]-malonsäure-bis-ethylester, 0,9 g (15 m Mol) Kaliumhydroxid, 30 ml Ethanol und 20 ml Wasser wird 15 Stunden unter Rückfluß zum Sieden erhitzt, dann auf etwa das halte Volumen eingeengt und mit konz. Salzsäure angesäuert. Das kristallin angefallene Produkt wird durch Absaugen isoliert.

Man erhält 0,4 g (15 % der Theorie) α-[2-Trifluormethylthio-5-(2,6-dichlor-4-trifluormethyl-phenoxy)-benzyl]-malonsäure vom Schmelzpunkt 250° C.

Beispiel 3

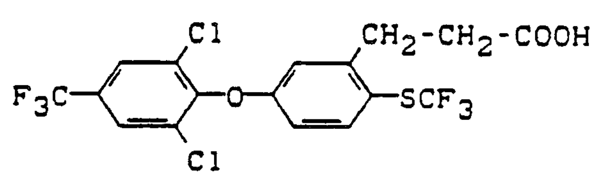

[Verfahren (c)]

2,7 g (5,2 m Mol) α-[2-Trifluormethylthio-5-(2,6-dichlor-4-trifluormethyl-phenoxy)-benzyl]-malonsäure

werden 4 Stunden auf 160 °C bis 170 °C erhitzt. Nach Abkühlen erhalt man 1,6 g (64 % der Theorie) β-[2-Trifluormethylthio-5-(2,6-dichlor-4-trifluormethyl-phenoxy)-phenyl]-propionsäure als kristallinen Rückstand vom Schmelzpunkt 131 °C.

Beispiel 4

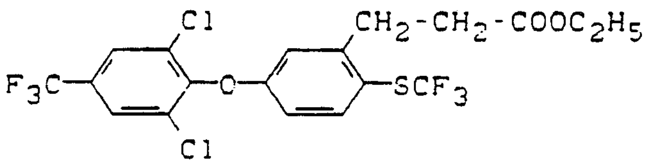

[Verfahren (f)

Eine Mischung aus 1,0 g (2 m Mol) β-[2-Trifluormethylthio-5-(2,6-dichlor-4-trifluormethyl-phenoxy)-phenyl]-propionsäure, 80 ml Ethanol und 1 ml konz. Schwefelsäure wird 20 Stunden unter Rückfluß zum Sieden erhitzt und dann eingeengt. Der Rückstand wird in Chloroform aufgenommen, diese Lösung mit Wasser und mit gesättigter Natriumbicarbonatlösung gewaschen, mit Natriumsulfat getrocknet und filtriert. Vom Filtrat wird das Lösungsmittel im Wasserstrahlvakuum sorgfältig abdestilliert.

Man erhält 0,6 g (60 % der Theorie) β-[2-Trifluormethylthio-5-(2,6-dichlor-4-trifluormethyl-phenoxy)-phenyl]-propionsäure-ethylester als amorphen Rückstand.

Analog zu den Beispielen 1 bis 4 und entsprechend der allgemeinen Beschreibung der erfindungsgemäßen Herstellungsverfahren können die in der nachstehenden Tabelle 4 aufgeführten Verbindungen der Formel (I) hergestellt werden.

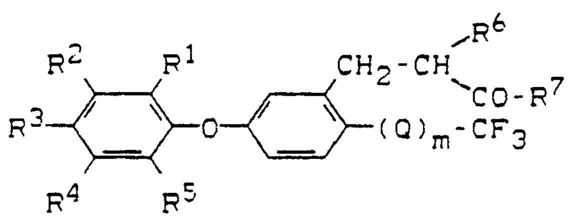

(I)

Tabelle 4

| Beispiele für die Verbindungen der Formel (I) | | | | | | | | | | |
|---|---|---|---|---|---|---|---|---|---|---|
| Bsp.-Nr. | $R^1$ | $R^2$ | $R^3$ | $R^4$ | $R^5$ | $R^6$ | $R^7$ | Q | m | $^1$H-NMR oder Schmelzpunkt /°C |
| 5 | Cl | H | $CF_3$ | H | Cl | COOH | OH | - | 0 | 138 |
| 6 | Cl | H | $CF_3$ | H | Cl | H | OH | - | 0 | 165 |
| 7 | Cl | H | $CF_3$ | H | Cl | H | $OC_2H_5$ | - | 0 | $^1$H-NMR*: 1,25/2,60/3,10/4,13 |
| 8 | Cl | H | $CF_3$ | H | Cl | H | OH | $SO_2$ | 1 | |
| 9 | Cl | H | $CF_3$ | H | Cl | COOH | OH | $SO_2$ | 1 | 195 |
| 10 | Cl | H | $CF_3$ | H | Cl | $COOC_2H_5$ | $OC_2H_5$ | $SO_2$ | 1 | |
| 11 | Cl | H | $CF_3$ | H | Cl | $COOC_2H_5$ | $OC_2H_5$ | - | 0 | |

* Die $^1$H-NMR-Spektren wurden in Deuterochloroform ($CDCl_3$) mit Tetramethylsilan (TMS) als inneren Standard aufgenommen, Angegeben ist die chemische Verschiebung als δ wert in pm

Ausgangsstoffe der Formel (II)

Beispiel (II-1)

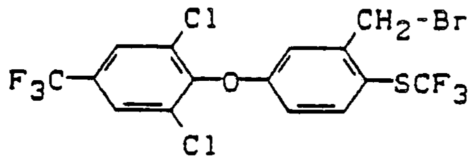

Eine Mischung aus 16,5 g (0,04 Mol) 2-Trifluormethylthio-5-(2,6-dichlor-4-trifluormethyl-phenoxy)-toluol 7,2 g (0,04 Mol) N-Brom-succinimid, 100 ml Tetrachlormethan und einer Spatelspitze Benzoylperoxid wird 48 Stunden unter Rückfluß zum Sieden erhitzt und anschließend filtriert. Vom Filtrat wird das Lösungsmittel im Wasserstrahlvakuum sorgfältig abdestilliert.

Man erhält 16,6 g (83 % der Theorie) 2-Trifluormethylthio-5-(2,6-dichlor-4-trifluormethyl-phenoxy)-benzylbromid als amorphen Rückstand.

Analog erhält man:

Beispiel (II-2)

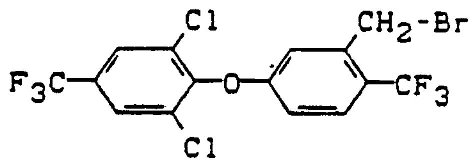

Beispiel (II-3)

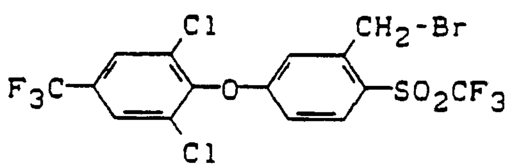

Ausgangsstoffe der Formel (VI)

Beispiel (VI-1)

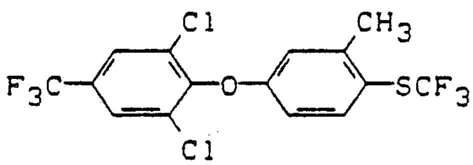

42 g (1,05 Mol) Natriumhydroxid-Pulver werden unter Rühren zu einer Lösung von 195 g (0,94 Mol) 3-Methyl-4-trifluormethylthio-phenol in 1,0 l Dimethylformamid gegeben, das Gemisch wird dann 30 Minuten bei 25° C bis 40° C gerührt, mit 100 ml Toluol verdünnt und anschließend so lange Lösungsmittel-Wasser-Gemisch abdestilliert, bis eine Innentemperatur von 130° C erreicht ist Dann werden 245 g (1,05 Mol) 3,5-Dichlor-4-fluor-benzotrifluorid dazugegeben und das Reaktionsgemisch wird 10 Stunden bei 125° C gerührt. Das Lösungsmittel wird dann bei 10 bis 10 mbar weitgehend abdestilliert.

Der Rückstand wird mit einem Zweiphasengemisch aus 300 ml Wasser und 400 ml Toluol verrührt. Die organische Phase wird abgetrennt, mit Natriumsulfat getrocknet und filtriert.

Das Filtrat wird durch Destillation unter vermindertem Druck aufgearbeitet. Als Hauptfraktion erhält man 278 g Rohprodukt vom Siedebereich 125° C bis 133° C (bei 0,1 mbar), welches bei 20° C allmählich durchkristallisiert. Nach Abpressen auf Ton erhält man 245 g (62 % der Theorie) 2-Trifluormethylthio-5-(2,6-dichlor-4-trifluormethyl-phenoxy)-toluol vom Schmelzpunkt 57° C bis 58° C.

Beispiel (VI-2)

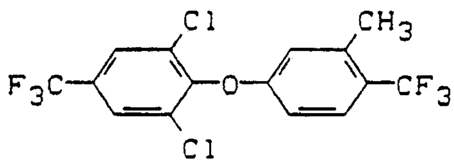

In einem VA-Autoklaven werden 750 ml Hydrogenfluorid (wasserfrei) bei 0° C bis 10° C vorgelegt, und eine Mischung aus 150 g (0,5 Mol) 3-(2,6-Dichlor-4-trifluormethyl-phenoxy)-toluol und 750 ml Tetrachlormethan wird dazugegeben. Das Gemisch wird unter einem Stickstoffdruck von 3 bar auf 115° C erhitzt. Entstehendes Hydrogenchlorid wird über einen Kühler mit automatischem Druckhalteventil bei 25 bar entspannt. Nach 7 Stunden bei 115° C wird abgekühlt und das überschüssige Hydrogenfluorid destillativ zurückgewonnen. Der Rückstand wird mit Wasser gewaschen und destilliert. Bei einem Siedebereich von 110° C bis 114° C (0,05 mbar) erhält man 118 g eines Produktgemisches, welches nach gaschromatischer Analyse 67,2 % 2-Trifluormethyl-5-(2,6-dichlor-4-trifluormethyl-phenoxy)-toluol enthält.

Beispiel (VI-3)

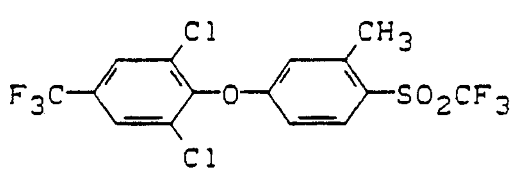

Eine Mischung aus 20 g (0,05 Mol) 2-Trifluormethylthio-5-(2,6-dichlor-4-trifluormethyl-phenoxy)-toluol und 100 ml Essigsäure wird auf eine Temperatur zwischen 80° C und 90° C gebracht und bei dieser Temperatur werden 50 ml 30 %iges wässriges Hydrogenperoxid tropfenweise dazugegeben. Das Reaktionsgemisch wird nach 3 Stunden bei 100° C gerührt, dann auf 20° C abgekühlt und in 250 ml Wasser eingerührt.

Das hierbei kristallin angefallene Produkt wird durch Absaugen isoliert.

Man erhält 20 g (93 % der Theorie) 2-Trifluormethylsulfonyl-5-(2,6-dichlor-4-trifluormethyl-phenoxy)-toluol vom Schmelzpunkt 121 °C bis 122 °C.

Anwendungsbeispiele

In den folgenden Anwendungsbeispielen wird die nachstehend aufgeführte Verbindung als Vergleichssubstanz herangezogen:

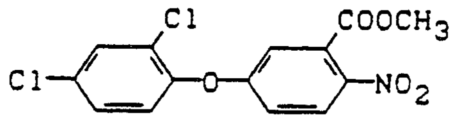

(A)

3-(2,4-Dichlor-phenoxy)-6-nitrobenzoesäure-methylester (bekannt aus US-PS 36 52 645 und US-PS 3 776 715).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt z.B. die Verbindung gemäß dem Herstellungsbeispiel 7 eine sehr gute selektive Bekämpfung von mono- und dikotylen Unkräutern insbesondere in monokotylen Kulturen wie beispielsweise Weizen.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit, insbesondere bei der Bekämpfung von dikotylen Unkräutern, gegenüber der Vergleichssubstanz (A) zeigt in diesem Test z. B. die Verbindung gemäß Herstellungsbeispiel 7.

## Ansprüche

1. Substituierte Phenoxyphenyl-propionsäure-Derivate der Formel (I)

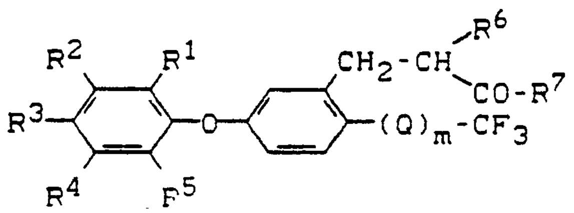

(I)

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Cyano, Carboxy, für gegebenenfalls durch Halogen substituiertes Alkyl oder für Alkoxycarbonyl steht,
$R^7$ für Halogen, Hydroxy, Amino, Alkylamino, Alkenylamino, Alkinylamino, Arylamino, Aralkylamino, Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^8$ steht, worin
$R^8$ für einen gegebenenfalls durch Halogen substituierten Rest aus der Reihe Alkyl Alkenyl, Alkinyl, Alkoxyalkyl, Alkylthioalkyl , Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-Äquivalent steht
oder für die Gruppierung

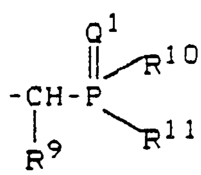

steht, worin
$R^9$ für Wasserstoff, Alkyl, Aryl, Furyl, Thienyl oder Pyridyl steht,
$R^{10}$ für Alkyl oder Alkoxy steht,
$R^{11}$ für Alkoxy steht und
$Q^1$ für Sauerstoff oder Schwefel steht, oder
$R^8$ für die Gruppierung $-(CH_2)_n-R^{12}$ steht, worin
n für die Zahlen 0, 1 oder 2 steht und
$R^{12}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl , Oxazolyl, Thiazolyl, Thiadiazolyl , Dioxolanyl, Perhydropyrrolyl, Oxoperhydropyrrolyl, Pyridinyl oder Pyrimidinyl steht.

2. Substituierte Phenoxyphenylpropionsäure-Derivate der Formel (I) gemäß Anspruch 1, in welcher
Q für S, SO oder $SO_2$ steht,
m für die Zahlen 0 oder 1 steht,
$R^1$ für Wasserstoff, Halogen, Cyano oder Trifluormethyl steht,

$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Cyano, Carboxy, für gegebenenfalls durch Fluor und/oder Chlor substituiertes $C_1$-$C_4$-Alkyl oder für $C_1$-$C_4$-Alkoxycarbonyl steht,
$R^7$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxy-carbonyl-$C_1$-$C_2$-alkylamino, Cyanoamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung O-$R^8$ steht, worin
$R^8$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl oder für ein Ammonium-, ein $C_1$-$C_4$-Alkylammonium-, ein Natrium-, Kalium-oder Calcium-Äquivalent steht, oder für die Gruppierung

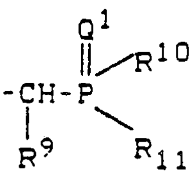

steht, worin
$R^9$ für Wasserstoff, $C_1$-$C_4$-Alkyl, Phenyl, Furyl, Thienyl oder Pyridyl steht,
$R^{10}$ für $C_1$-$C_4$-Alkyl oder $C_1$-$C_4$-Alkoxy steht,
$R^{11}$ für $C_1$-$C_4$-Alkoxy steht und
$Q^1$ für Sauerstoff oder Schwefel steht, oder
$R^8$ für die Gruppierung $-(CH_2)_n-R^{12}$ steht, worin
n für die Zahlen 0, 1 oder steht und
$R^{12}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

3. Verfahren zur Herstellung von substituierten Phenoxyphenylpropionsäure-Derivaten der Formel (I)

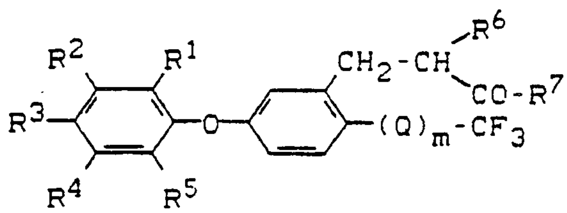

(I)

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Cyano, Carboxy, für gegebenenfalls durch Halogen substituiertes Alkyl oder für Alkoxycarbonyl steht,
$R^7$ für Halogen, Hydroxy, Amino, Alkylamino, Alkenylamino, Alkinylamino, Arylamino, Aralkylamino,

Alkoxycarbonyl-alkylamino, Cyanoamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonyl-alkylthio oder für die Gruppierung O-$R^8$ steht, worin

$R^8$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonyl alkyl, Aryloxyalkyl, Trialkkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl oder für ein Ammonium-, Alkylammonium-, Alkalimetall- oder Erdalkalimetall-Äquivalent steht oder für die Gruppierung

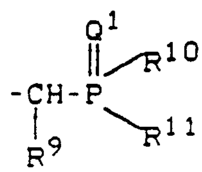

steht, worin

$R^9$ für Wasserstoff, Alkyl, Aryl, Furyl, Thienyl oder Pyridyl steht,

$R^{10}$ für Alkyl oder Alkoxy steht,

$R^{11}$ für Alkoxy steht und

$Q^1$ für Sauerstoff oder Schwefel steht, oder

$R^8$ für die Gruppierung $-(CH_2)_n-R^{12}$ steht, worin

n für die Zahlen 0, 1 oder 2 steht und

$R^{12}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,

dadurch gekennzeichnet, daß man

(a) substituierte Phenoxybenzylhalogenide der allgemeinen Formel (II)

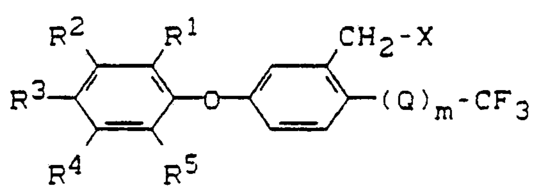

(II)

in welcher

Q, $R^1$, $R^2$, $R^3$, $R^4$, $R^5$ und m die oben angegebenen Bedeutungen haben und

X für Halogen steht,

mit Carbonylverbindungen der allgemeinen Formel (III)

$R^6 - CH_2 - CO - R^7$ (III)

in welcher

$R^6$ und $R^7$ die oben angegebenen Bedeutungen haben,

gegebenenfalls in Gegenwart eines Säureakzeptors und gegebenenfalls in Gegenwart eines Verdünnungsmittels umsetzt, oder daß man

(b) für den Fall, daß in Formel (I)

$R^6$ für Carboxy steht und

$R^7$ für Hydroxy steht sowie

Q, $R^1$, $R^2$, $R^3$, $R^4$, $R^5$ und m die oben angegebenen Bedeutungen haben,

Verbindungen der allgemeinen Formel (I), in welcher

$R^6$ für Alkoxycarbonyl steht und

$R^7$ für Alkoxy steht sowie

Q, $R^1$, $R^2$, $R^3$, $R^4$, $R^5$ und m die oben angegebenen Bedeutungen haben,

mit Wasser, gegebenenfalls in Gegenwart eines Verseifungshilfsmittels und gegebenenfalls in Gegenwart eines Verdünnungsmittels umsetzt, oder daß man

(c) für den Fall, daß in Formel (I)

$R^6$ für Wasserstoff steht und

$R^7$ für Hydroxy steht sowie

Q, $R^1$, $R^2$, $R^3$, $R^4$, $R^5$ und m die oben angegebenen Bedeutungen haben,
Verbindungen der allgemeinen Formel (I), in welcher
$R^6$ für Carboxy steht und
$R^7$ für Hydroxy steht sowie
Q, $R^1$, $R^2$, $R^3$, $R^4$, $R^5$ und m die oben angegebenen Bedeutungen haben,
pyrolytisch decarboxyliert, oder daß man

(d) für den Fall, daß in Formel (I)
$R^6$ für Wasserstoff steht und
$R^7$ für Halogen steht sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Wasserstoff steht und
$R^7$ für Hydroxy steht sowie
Q, $R^1$, $R^2$, $R^3$, $R^4$, $R^5$ und m die oben angegebenen Bedeutungen haben,
mit einem Halogenierungsmittel, gegebenenfalls in Gegenwart eines Katalysators und gegebenenfalls in Gegenwart eines organischen Lösungsmittels umsetzt, oder daß man

(e) für den Fall, daß in Formel (I)
$R^6$ für Wasserstoff steht und
$R^7$ mit Ausnahme von Halogen die oben angegebene Bedeutung hat sowie
Q, $R^1$, $R^2$, $R^3$, $R^4$, $R^5$ und m die oben angegebenen Bedeutungen haben,
Verbindungen der allgemeinen Formel (I), in welcher
$R^6$ für Wasserstoff steht und
$R^7$ für Halogen steht sowie
Q, $R^1$, $R^2$, $R^3$, $R^4$, $R^5$ und m die oben angegebenen Bedeutungen haben,
mit Verbindungen der Formel (IV)
H - $R^7$ (IV)
in welcher
$R^7$ mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder daß man

(f) für den Fall, daß in Formel (I)
$R^6$ für Wasserstoff steht und
$R^7$ für Alkoxy steht sowie
Q, $R^1$, $R^2$, $R^3$, $R^4$, $R^5$ und m die oben angegebenen Bedeutungen haben,
Verbindungen der allgemeinen Formel (I) in welcher
$R^6$ für Wasserstoff steht und
$R^7$ für Hydroxy steht sowie
Q, $R^1$, $R^2$, $R^3$, $R^4$, $R^5$ und m die oben angegebenen Bedeutungen haben,
mit Alkoholen der Formel (V)
HOR (V)
in welcher
R für Alkyl steht
gegebenenfalls in Gegenwart eines Katalysators umsetzt.

4. Herbizide Mittel, gekennzeichnet durch einen Gehalt an mindestens einem substituierten Phenoxyphenylpropionsäure-Derivat der Formel (I) gemäß den Ansprüchen 1 bis 3.

5. Verfahren zur Bekämpfung von Unkräutern, dadurch gekennzeichnet, daß man substituierte Phenoxyphenylpropionsäure-Derivate der Formel (I) gemäß den Ansprüchen 1 bis 3 auf die Unkräuter und/oder ihren Lebensraum einwirken läßt.

6. Verwendung von substituierten Phenoxyphenylpropionsäure-Derivaten der Formel (I) gemäß den Ansprüchen 1 bis 3 zur Bekampfung von Unkräutern.

7. Verfahren zur Herstellung von herbiziden Mitteln, dadurch gekennzeichnet, daß man substituierte Phen oxyphenylpropionsäure-Derivate der Formel (I) gemäß den Ansprüchen 1 bis 3 mit Streckmitteln und/oder oberflächenaktiven Substanzen vermischt.

8. Substituierte Phenoxybenzylhalogenide der Formel (II)

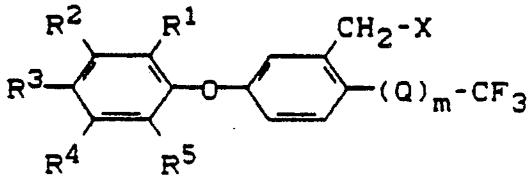

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht und
X für Halogen steht.

9. Substituierte Phenoxytoluole der Formel (VI)

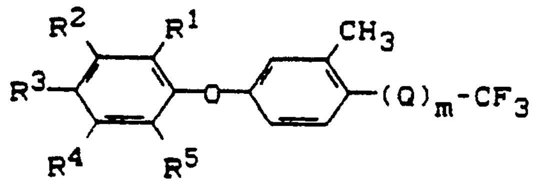

(VI)

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht und
$R^5$ für Wasserstoff oder Halogen steht.

10. Verfahren zur Herstellung von substituierten Phenoxybenzylhalogeniden der Formel (II)

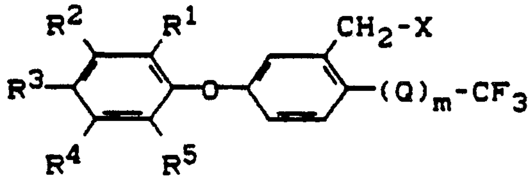

(II)

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht und
X für Halogen steht,
dadurch gekennzeichnet, daß man substituierte Phenoxytoluole der Formel (VI)

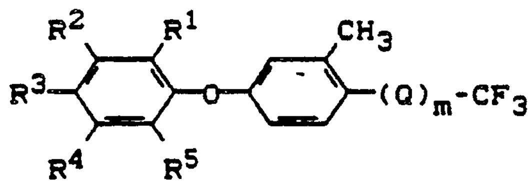

(VI)

in welcher
Q, $R^1$, $R^2$, $R^3$, $R^4$, $R^5$ und m die oben angegebenen Bedeutungen haben,
mit Halogenierungsmitteln gegebenenfalls in Gegenwart von Katalysatoren und gegebenenfalls in Gegenwart von Verdünnungsmitteln umsetzt.

11. Verfahren zur Herstellung von substituierten Phenoxytoluolen der Formel (VI)

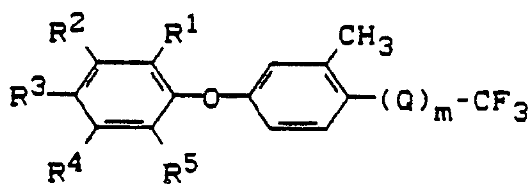

(VI)

in welcher
Q für S, SO oder $SO_2$ steht,
m für die Zahlen 0 oder 1 steht,
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht und
$R^5$ für Wasserstoff oder Halogen steht
dadurch gekennzeichnet, daß man Halogen-benzol-Derivate der Formel (VII)

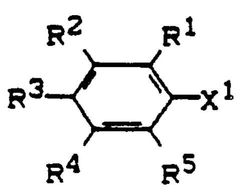

(VII)

in welcher
$R^1$, $R^2$, $R^3$, $R^4$ und $R^5$ die oben angegebenen Bedeutungen haben und
$X^1$ für Fluor oder Chlor steht,
mit Phenolderivaten der allgemeinen Formel (VIII)

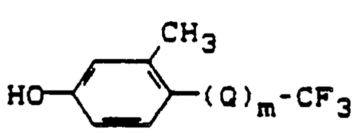

(VIII)

in welcher
Q und m die oben angegebenen Bedeutungen haben
in Gegenwart eines Säureakzeptors und in Gegenwart eines Verdünnungsmittels umsetzt,
oder daß man, für den Fall, daß in Formel (VI) m für die Zahl 0 steht, Phenoxytoluole der Formel (IX)

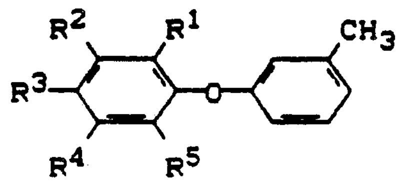

(IX)

in welcher
$R^1$, $R^2$, $R^3$, $R^4$ und $R^5$ die oben angegebenen Bedeutungen haben,
mit Hydrogenfluorid/Tetrachlormethan umsetzt,
oder daß man, für den Fall, daß in Formel (VI) m für die Zahl 1 und Q für SO oder $SO_2$ steht,
die Verbindungen der Formel (VI), in denen m für die Zahl 1 und Q für Schwefel steht, mit Oxidationsmitteln in Gegenwart eines Verdünnungsmittels umsetzt.

European Patent Office

# EUROPEAN SEARCH REPORT

Application number

EP 90102525.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| A | DE - A1 - 3 308 374 (VELSICOL CHEMICAL) * Ansprüche 1,9,11 * -- | 1,4-6 | C 07 C 323/62<br>C 07 C 317/44<br>C 07 C 59/68<br>C 07 C 69/65<br>C 07 C 323/20<br>C 07 C 43/29<br>C 07 C 317/22<br>A 01 N 37/38 |
| A | EP - A2 - 0 075 377 (IMPERIAL CHEMICAL INDUSTRIES) * Ansprüche 1-3 * -- | 1,4-6 | |
| A | EP - A2 - 0 138 183 (F.HOFFMANN-LA ROCHE) * Ansprüche 1,15,18,20 * -- | 1,4-6 | |
| A | DE - A - 1 941 625 (ELI LILLY) * Anspruch 1; Seiten 19 IId-20 IIe * -- | 1 | |
| A | US - A - 4 377 713 (KESTUTIS A.KEBLYS) * Zusammenfassung * -- | 8,10 | **TECHNICAL FIELDS SEARCHED (Int. Cl.⁵)** |
| A | US - A - 4 484 008 (JAMES A.COOK JR. et al.) * Spalte 1-Spalte 2, Zeile 4 * -- | 9 | C 07 C 323/00<br>C 07 C 317/00<br>C 07 C 59/00<br>C 07 C 69/00<br>C 07 C 43/00 |
| A | EP - A1 - 0 056 119 (BASF) * Ansprüche 3,4; Seiten 6,9 * ---- | 1,4-11 | |

The present search report has been drawn up for all claims

| Place of search | Date of completion of the search | Examiner |
|---|---|---|
| WIEN | 25-05-1990 | REIF |

CATEGORY OF CITED DOCUMENTS

- X particularly relevant if taken alone
- Y particularly relevant if combined with another document of the same category
- A technological background
- O non-written disclosure
- P intermediate document
- T : theory or principle underlying the invention
- E : earlier patent document, but published on, or after the filing date
- D : document cited in the application
- L : document cited for other reasons
- & : member of the same patent family, corresponding document